(19) Europäisches Patentamt
European Patent Office
Office européen des brevets

(11) **EP 4 382 158 A1**

(12) **EUROPEAN PATENT APPLICATION**

(43) Date of publication:
**12.06.2024 Bulletin 2024/24**

(21) Application number: **23213865.1**

(22) Date of filing: **04.12.2023**

(51) International Patent Classification (IPC):
***A61M 25/01*** (2006.01)

(52) Cooperative Patent Classification (CPC):
**A61M 25/0113;** A61M 25/013

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.12.2022 US 202218061648**

(71) Applicant: **Biosense Webster (Israel) Ltd.**
**2066717 Yokneam (IL)**

(72) Inventors:
• **SIDDIQUI, Masood**
**Irvine, 92618 (US)**
• **CORBIN, Matthew**
**Irvine, 92618 (US)**
• **BROOME, Thomas**
**Irvine, 92618 (US)**

(74) Representative: **Carpmaels & Ransford LLP**
**One Southampton Row**
**London WC1B 5HA (GB)**

(54) **CATHETER SHAFT GRIP APPARATUS**

(57)     An apparatus 200 to a grip a catheter shaft including a body including a proximal end extending along a central axis to a distal end. The body includes a first handle portion 202A, a second handle portion 202B, and a catheter shaft grip aperture 208. The first handle portion defines a first recess 212A extending along the central axis of the body from the proximal end to the distal end. The second handle portion defines a second recess 212B extending along the central axis of the body from the proximal end to the distal end such that the first recess is aligned with the second recess. The catheter shaft grip aperture extends along the central axis of the body from the proximal end to the distal end, is formed from the alignment of the first recess and the second recess, and is sized to receive a catheter shaft.

FIG. 2A

EP 4 382 158 A1

FIG. 2B

**Description**

FIELD

The present invention relates generally to medical devices, and in particular catheter shaft grip systems and methods.

BACKGROUND

Catheters are widely used in medical procedures. In many procedures, a user (e.g., a physician) must finely control the catheter during the procedure. Fine control of the catheter often occurs directly on the shaft of the catheter. For example, the user will grip the catheter shaft to manipulate (e.g., by rotation, push/pull) the catheter to move, rotate, and/or navigate the catheter and/or another device attached to the catheter. Because of the small diameter and smooth texture of a catheter shaft, the user is required to squeeze the shaft tightly in order to rotate the tip and to move the catheter in and out to vary the depth. This can be tiresome and can create hand cramping. Further, due to the small diameter and smooth texture of a catheter shaft, it can be difficult to finely control the catheter's movement. What is needed, therefore, are devices and methods for gripping a catheter shaft that can help users grip the catheter shaft, increase comfort, reduce fatigue and/or cramping, and increase fine control over the catheter.

SUMMARY

The disclosed technology can include an apparatus to grip a catheter shaft. The apparatus can include a body including a proximal end extending along a central axis to a distal end. The body can include a first handle portion, a second handle portion, and a catheter shaft grip aperture. The first handle portion can define a first recess extending along the central axis of the body from the proximal end to the distal end. The second handle portion can define a second recess extending along the central axis of the body from the proximal end to the distal end such that the first recess is aligned with the second recess. The catheter shaft grip aperture can extend along the central axis of the body from the proximal end to the distal end. The catheter shaft grip aperture can be formed from the alignment of the first recess and the second recess. The catheter shaft grip aperture can be sized to receive a catheter shaft.

The catheter shaft grip aperture can frictionally engage a catheter shaft and transfers rotational force applied to the body to a catheter shaft.

The catheter shaft grip aperture can include a relaxed state and a grip state. The relaxed state can be where the catheter shaft grip aperture receives a catheter shaft and is freely movable along a catheter shaft. The grip state can be where a force applied to the body causes the catheter shaft grip aperture to frictionally engage a catheter shaft.

The first handle portion can include a first connector extending outwardly from the first handle portion. The second handle portion can include a second connector configured to receive the first connector and attach the first handle portion and the second handle portion to each other.

The first connector can include a first male connector disposed proximate the proximal end. The second connector can include a first female connector disposed proximate the proximal end. The first handle portion can include a second male connector extending outwardly from the first handle portion and disposed proximate the distal end. The second handle portion can include a second female connector disposed proximate the distal end and configured to receive the second male connector to attach the first handle portion and the second handle portion to each other.

The first handle portion can include a first hinge portion and the second handle portion including a second hinge portion. The first hinge portion and the second hinge portion can be rotatably connected to each other such that the first handle portion and the second handle portion can be rotated between an engaged configuration and a disengaged configuration.

The first handle portion can include a second recess. The second recess can extend into the first handle portion generally perpendicularly to the central axis. The second handle portion can include a protrusion extending outwardly from second handle portion generally perpendicularly to the central axis. The second recess can be configured to receive the protrusion to facilitate alignment of the first handle portion with the second handle portion.

The apparatus can include a retention member. The retention member can be configured to apply a force to a catheter shaft when the retention member is in an engaged position.

The retention member can be rotatable between the engaged position and a disengaged position.

The retention member can include a lever arm. The level arm can include an engagement end configured to engage a catheter shaft. The lever arm can be configured to be actuated between the engaged position and the disengaged position by a user pushing on the lever arm at an end opposite the engagement end.

The retention member can be biased to remain in the engaged position.

The retention member can be spring-loaded.

The body can include a proximal attachment end. The proximal attachment end can be configured to attach to a handle device of a catheter assembly.

The attachment end can include a threaded end.

The body can include a distal attachment end. The distal end can be configured to attach to a valve of a catheter assembly.

The first recess and the second recess can each include a textured inner surface configured to engage a catheter shaft.

The textured inner surface can include a plurality of ribs extending along the inner surface.

The textured inner surface can include a knurled finish.

The body can include a textured outer surface. The textured outer surface can be configured to be gripped by a user of the apparatus.

The textured outer surface can include a plurality of ribs extending along the outer surface.

The textured outer surface can include a knurled finish.

The body can include a diameter of approximately one inch.

The aperture can include a diameter of approximately 0.1 inches.

The inner diameter of the aperture can be less than an outer diameter of a catheter shaft.

The aperture can form a friction fit with a catheter shaft.

The body can include an ergonomic outer shape. The ergonomic outer shape can be configured to conform to a user's hand.

The body can include a tip proximate the distal end. The tip can be configured to serve as an insertion tool.

The disclosed technology can include a handle for gripping a catheter shaft. The handle can include a first handle portion and a second handle portion. The first handle portion can define a first channel extending from a proximal end to a distal end of the first handle portion. The second handle portion can define a second channel extending from a proximal end to a distal end of the second handle portion. The first handle portion and the second handle portion can be attachable to each other to form a handle including an aperture extending therethrough formed by the first channel and the second channel. The aperture can be sized to receive a catheter shaft.

The first handle portion can include a first connector extending outwardly from the first handle portion. The second handle portion can include a second connector. The second connector can be configured to receive the first connector and attach the first handle portion and the second handle portion to each other.

The first connector can include a first male connector disposed proximate the proximal end of the first handle portion. The second connector can include a first female connector disposed proximate the proximal end of the second handle portion. The first handle portion can include a second male connector extending outwardly from the first handle portion and disposed proximate the distal end of the first handle portion. The second handle portion can include a second female connector disposed proximate the distal end of the second handle portion. The second female connector can be configured to receive the second male connector to attach the first handle portion and the second handle portion to each other.

The first handle portion can include a second recess extending into the first handle portion generally perpendicularly to a central axis of the first handle portion. The second handle portion can include a protrusion extending outwardly from second handle portion generally perpendicularly to a central axis of the second handle portion. The second recess can be configured to at least partially receive the protrusion to facilitate alignment of the first handle portion with the second handle portion.

The first handle portion can include a first hinge portion. The second handle portion can include a second hinge portion. The first hinge portion and the second hinge portion can be rotatably connected to each other such that the first handle portion and the second handle portion can be rotated between an engaged configuration and a disengaged configuration.

The first connector can be disposed on a side of the first handle portion opposite the first hinge portion. The second connector can be disposed on a side of the second handle portion opposite the second hinge portion such that the second connector is configured to receive the first connector and secure the first handle portion to the second handle portion when in the engaged configuration.

The disclosed technology can include a method of manipulating a catheter shaft using an attachable handle. The method can include aligning a first handle portion with a catheter shaft. The method can include inserting a catheter shaft into a first recess of the first handle portion. The first recess can extend along a central axis of the first handle portion. The method can include aligning a second handle portion with the first handle portion. The method can include attaching the second handle portion to the first handle portion to form a handle. The second handle portion can include a second recess extending along a central axis of the second handle portion such that the first recess and the second recess form an aperture through the handle. The aperture can be sized to receive a catheter shaft. The method can include gripping the handle and manipulating a catheter shaft by rotating the handle.

Additional features, functionalities, and applications of the disclosed technology are discussed herein in more detail.

BRIEF DESCRIPTION OF THE DRAWINGS

FIG. 1 illustrates a schematic pictorial illustration of a medical system including a medical probe, in accordance with an example of the disclosed technology;
FIG. 2A is a perspective view of a catheter shaft grip apparatus, in accordance with an example of the disclosed technology;
FIG. 2B illustrates an exploded view of the catheter shaft grip apparatus of FIG. 2A, in accordance with an example of the disclosed technology;
FIG. 2C illustrates an elevation view and detail view of the catheter shaft grip apparatus of FIG. 2A, in accordance with an example of the disclosed technology;
FIG. 3 illustrates an elevation view of a catheter shaft grip apparatus, in accordance with an example of the disclosed technology;
FIG. 4 illustrates a section view of a catheter shaft grip apparatus, in accordance with an example of the disclosed technology;
FIG. 5 illustrates a method of using a catheter shaft grip apparatus, in accordance with an example of the disclosed technology;

DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several examples, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values $\pm 20\%$ of the recited value, e.g., "about 90%" may refer to the range of values from 71% to 110%.

As used herein, the terms "patient," "host," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" or "user" can include a doctor, surgeon, technician, scientist, or any other individual or delivery instrumentation associated with delivery of a catheter to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

The present disclosure is related to systems, methods or uses and devices which utilize a catheter shaft grip apparatus. Example systems, methods, and devices of the present disclosure may be particularly suited for catheters for IRE ablation of cardiac tissue to treat cardiac arrhythmias. Ablative energies are typically provided to cardiac tissue by an end portion of a catheter which can deliver ablative energy to the tissue to be ablated. Some example catheters include three-dimensional structures at the end portion and are configured to administer ablative energy from various electrodes positioned on the three-dimensional structures. Ablative procedures incorporating such example catheters can be visualized using fluoroscopy.

Although certain examples of the disclosed technology are explained in detail, it is to be understood that other examples, embodiments, and implementations of the disclosed technology are contemplated. Accordingly, it is not intended that the disclosed technology is limited in its scope to the details of construction and arrangement of components set forth in the following description or illustrated in the drawings. The disclosed technology can be implemented in a variety of examples and can be practiced or carried out in various ways. In particular, the presently disclosed subject matter is described in the context of being for catheters for IRE ablation of cardiac tissue. The present disclosure, however, is not so limited, and can be applicable to other technologies. The present disclosure, for example and not limitation, can include additional systems, methods or uses and devices which utilize a catheter shaft grip apparatus including other medical, surgical, and veterinary procedures that utilize catheters. Such implementations and applications are contemplated within the scope of the present disclosure. Accordingly, when the present disclosure is described in the context of being for catheters for IRE ablation of cardiac tissue, it will be understood that other implementations can take the place of those referred to.

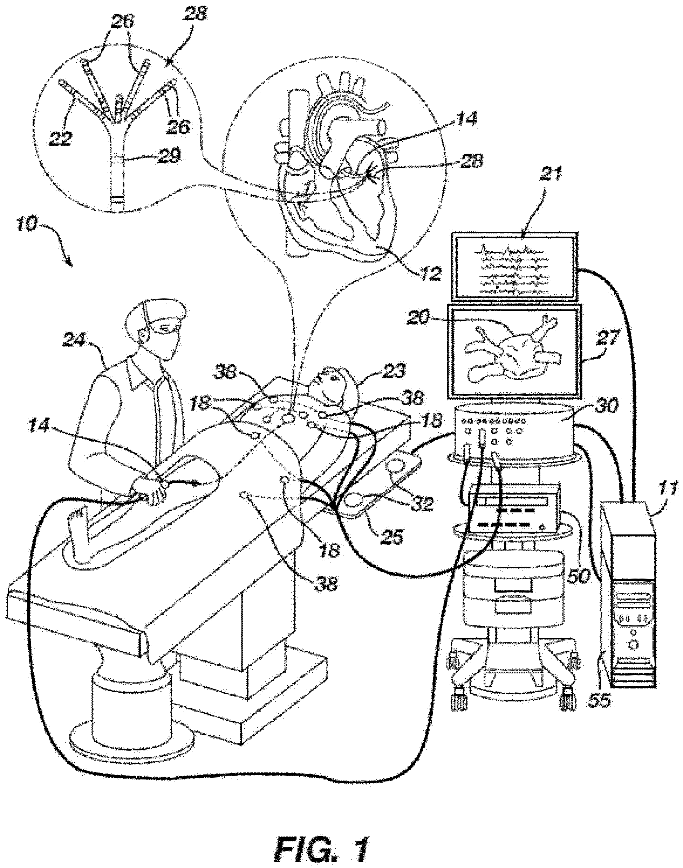

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip 28 of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO™ 3 System, available from Biosense Webster, Inc., 31A Technology Drive, Irvine, CA 92618.

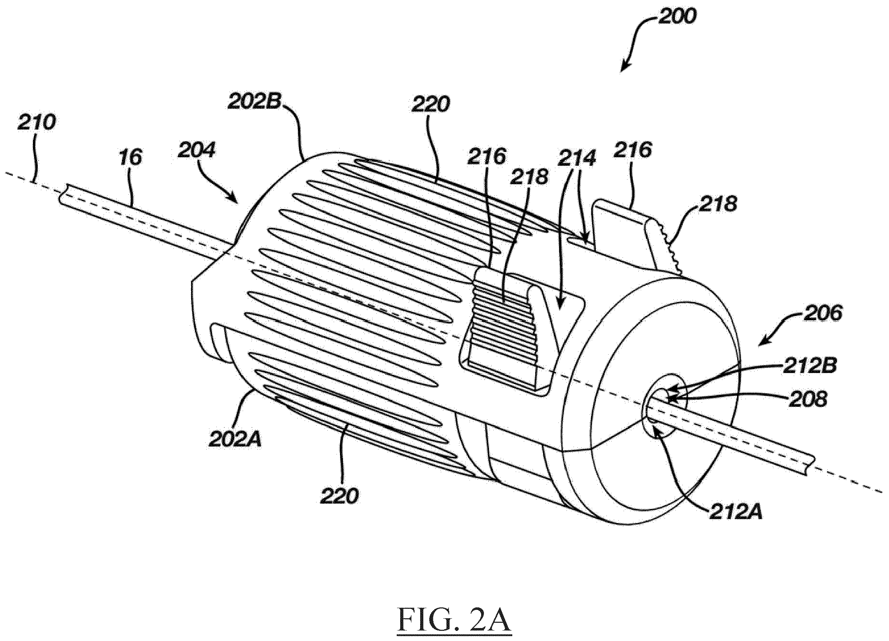

FIG. 2A is an illustration of a catheter shaft grip apparatus 200, in accordance with an example of the present invention. Catheter shaft grip apparatus 200 can include a first handle portion 202A and a second handle portion 202B. The first handle portion 202A and the second handle portion 202B can form the body of the catheter shaft grip apparatus 200. The first handle portion 202A can include a first recess 212A and the second handle portion 202B can include a second recess 212B. The recesses described herein (e.g., first recess 212A and second recess 212B) can also be described as channels. In the configuration shown in FIG. 2A, the first handle portion 202A is attached to the second handle portion 202B. When the first handle portion 202A is attached to the second handle portion 202B, the first recess 212A and the second recess 212B form a catheter shaft grip aperture 208 extending along the central axis 210. The catheter shaft grip aperture 208 can be sized to receive a catheter shaft 16. For example, the catheter shaft 16 of catheter 14. The catheter shaft grip aperture 208 can further be configured to frictionally engage the catheter shaft 16 such that when rotational force is applied to the body of the catheter shaft grip apparatus 200, the rotational force is transferred to the catheter shaft 16. For example, the inner diameter of the catheter shaft grip aperture 208 can be less than an outer diameter of a catheter shaft 16. Alternatively, the inner diameter of the catheter shaft grip aperture 208 can be the same as the outer diameter of a catheter shaft 16. As will be appreciated, during a medical procedure, a physician 24 can cause the catheter shaft 16 to rotate as desired by rotating the catheter shaft grip apparatus 200.

The catheter shaft grip apparatus 200 can include a distal end 204 and a proximal end 206. For example, during a medical procedure, catheter shaft grip apparatus 200 can be attached to a catheter shaft 16 and the proximal end can be disposed closer to a physician 24 and the distal end 204 can be disposed farther away from the physician 24.

The catheter shaft grip apparatus 200 can include connectors configured to attach the first handle portion 202A and the second handle portion 202B. For example, the first handle portion 202A can include a first connector 216. The first connector 216 can be disposed proximate the proximal end 206. The first connector 216 can be a protrusion that extends outwardly from the first handle portion 202A. Alternatively, the first connector 216 can include a plurality of protrusions extending outwardly from the first handle portion 202A. For example, the first connector 216 can include a first male connector and a second male connector. The second handle portion 202B can include a second connector 214. The second connector 214 can be disposed proximate the proximal end 206. The second connector 214 can be an opening and/or a recess configured to receive the first connector 216. Alternatively, the second connector 214 can include a plurality of openings and/or recesses configured to each receive one of a plurality of protrusions of the first connector 216. For example, the second connector 214 can include a first female connector and a second female connector. The first male connector of the first connector 216 and the first female connector of the second connector 214 can be configured to form a connection. Additionally, the second male connector of the first connector 216 and the second female connector of the second connector 214 can be configured to form a connection. For example, the first connector 216 and the second connector 214 can form a snap-fit connection. Alternatively, or in addition, the first connector 216 and the second connector 214 can form a connection using a different mechanism such as tabs, screws, adhesive, interference fit, and the like, or any combination thereof. As illustrated in FIG. 2A, the first connector 216 can include first and second male connector and the second connector 214 can include first and second female connector configured to receive the first and second male connectors of the first connector 216 and form a snap fit to attach the first handle portion 202A to the second handle portion 202B.

The first connector 216 can include a textured outer surface. For example, the textured outer surface can include a plurality of ribs 218 extending along the outer surface. Alternatively, or in addition, the textured outer surface can include a knurled finish and/or coined edge finish. The textured outer surface can be configured to assist with gripping, squeezing, and/or pushing the first connector 216. For example, the textured outer surface can provide grip so that it is easier for a user to pinch the first connector 216 to un-snap the snap-fit formed between the first connector 216 and the second connector 214.

The catheter shaft grip apparatus 200 can include a textured outer surface. For example, the textured outer surface can include a plurality of ribs 220 extending along the outer surface. Alternatively, or in addition, the textured outer surface can include a knurled finish and/or coined edge finish. The textured outer surface can be configured to assist with gripping, squeezing, and/or rotating the catheter shaft grip apparatus 200. For example, the textured outer surface can provide grip so that it is easier for a user to hold, rotate, and move the catheter shaft grip apparatus 200.

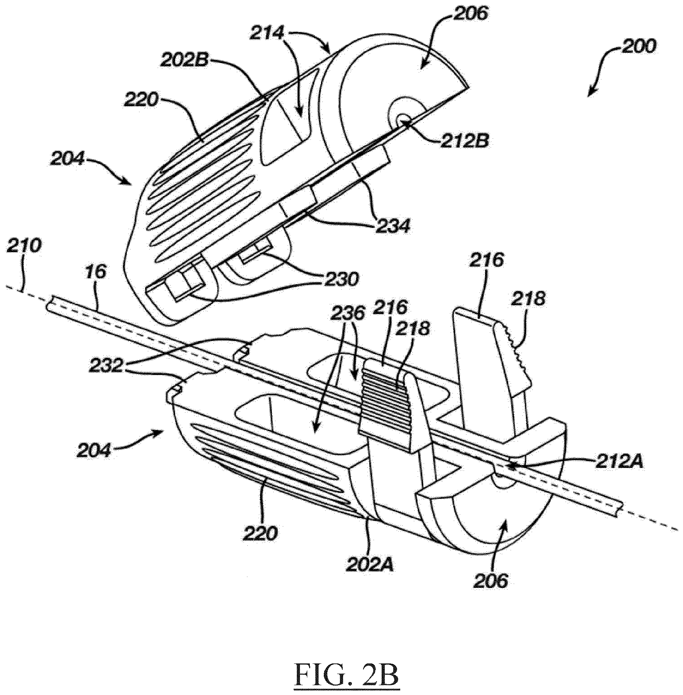

As illustrated in FIG. 2B, the first handle portion 202A and second handle portion 202B can be separate pieces. As explained herein, the first handle portion 202A and second handle portion 202B can be attached via the first connector 216 and the second connector 214 disposed proximate the proximal end 206. Alternatively, or in addition, the first handle portion 202A can include a third connector 232 and the second handle portion 202B can include a fourth connector 230. The third connector 232 and the fourth connector 230 can be disposed proximate the distal end 204. The third connector 232 and the fourth connector 230 can be configured to attach the first handle portion 202A and second handle portion 202B. The third connector 232 can be a protrusion that extends outwardly from the first handle portion 202A. Alternatively, the third connector 232 can include a plurality of protrusions extending outwardly from the first handle portion 202A. For example, the third connector 232 can include a first male connector and a second male connector. The second handle portion 202B can include a fourth connector 230. The fourth connector 230 can be an opening and/or a recess configured to receive the third connector 232. Alternatively, the fourth connector 230 can include a plurality of openings and/or recesses configured to each receive one of a plurality of protrusions of the third connector 232. For example, the fourth connector 230 can include a first female connector and a second female connector. The first male connector of the third connector 232 and the first female connector of the fourth connector 230 can be configured to form a connection. Additionally, the second male connector of the third connector 232 and the second female connector of the fourth connector 230 can be configured to form a connection. For example, the third connector 232 can be inserted into the fourth connector 230 such that the first handle portion 202A and second handle portion 202B are generally pivotally connected by the third connector 232 and fourth connector 230. Alternatively, or in addition, the third connector 232 and fourth connector 230 can form a connection using a different mechanism such as snap-fit, tabs, screws, adhesive, interference fit, hook-and-loop fasteners, live hinge, pin hinge, snap hinge, and the like, or any combination thereof.

As will be appreciated, the first handle portion 202A and second handle portion 202B can be attached at proximal end 206 by the first and second connectors 216, 214 and attached at the distal end 204 by the third and fourth connector 232, 230 such that the first handle portion 202A and second handle portion 202B are securely attached. The first handle portion 202A and second handle portion 202B can further be detached by disconnecting the first and second connector 216, 214 and/or disconnecting the third and fourth connectors 232, 230. For example, the first handle portion 202A and second handle portion 202B can be detached by pinching the first connector 216 such that it disengages from its snap-fit connection with the second connector 214.

The first handle portion 202A can further include one or more recesses 236 and the second handle portion 202B can include one or more protrusions 234. As illustrated in FIG. 2B, the first handle portion 202A can include two recesses 236 extending into the first handle portion 202A generally perpendicular to the central axis 210. The second handle portion 202B can include two protrusions 234 extending outwardly from the second handle portion 202B generally perpendicular to the central axis 210. The recesses 236 can be configured to receive the protrusions 234. As will be appreciated, mating the protrusions 234 to the recesses 236 can facilitate alignment of the first handle portion 202A with the second handle portion 202B.

As illustrated in FIG. 2B, the first recess 212A and the second recess 212B can extend along the central axis 210 from the proximal end 206 to the distal end 204. The first recess 212A and the second recess 212B can both be generally semicircular in profile such that when the first handle portion 202A and the second handle portion 202B are attached, the first recess 212A and the second recess 212B form a catheter shaft grip aperture 208 with a generally circular profile extending the length of the catheter shaft grip apparatus 200. Alternatively, the profile of the catheter shaft grip aperture 208 can be generally a different shape, such as a rectangle, hexagon, octagon, star, or another polygon. The catheter shaft grip aperture 208 can be sized to receive a catheter shaft 16.

The catheter shaft grip aperture 208 can have a relaxed state where the catheter shaft grip aperture 208 receives a catheter shaft 16 and is freely movable along a catheter shaft. For example, the relaxed state can be prior to the first handle portion 202A and the second handle portion 202B being snapped or otherwise connected together. Alternatively, the relaxed state can be when the first handle portion 202A and the second handle portion 202B are snapped or otherwise connected together and attached but no external force has been applied to the body of the catheter shaft grip apparatus 200. For example, the relaxed state can be when no pinching or squeezing force has been applied by a user to the body of the catheter shaft grip apparatus 200.

The catheter shaft grip aperture 208 can have a grip state where the catheter shaft grip aperture 208 frictionally engages the catheter shaft. For example, the grip state can be when the first handle portion 202A and the second handle portion 202B are attached. Alternatively, the grip state can be when an external force is applied to the body of the catheter shaft grip apparatus 200. For example, grip state can be when a pinching or squeezing force has been applied by a user to the body of the catheter shaft grip apparatus 200.

The catheter shaft grip aperture 108 can frictionally engage the catheter shaft 16 such that when a rotational force is applied to the body of the catheter shaft grip apparatus 200, the rotational force is transferred to the catheter shaft 16. For example, the catheter shaft grip aperture 108 can form a friction fit with the catheter shaft 16. The inner diameter of the catheter shaft grip aperture 108 can be less than an outer diameter of a catheter shaft 16. For example, the catheter shaft grip aperture 108 can have a diameter of approximately 0.1 inches. Alternatively, the catheter shaft grip aperture 108 can have a diameter less than 0.1 inches to receive smaller catheter shafts 16 as required. Alternatively, the catheter shaft grip aperture 108 can have a diameter greater than 0.1 inches to receive larger catheter shafts 16 as required.

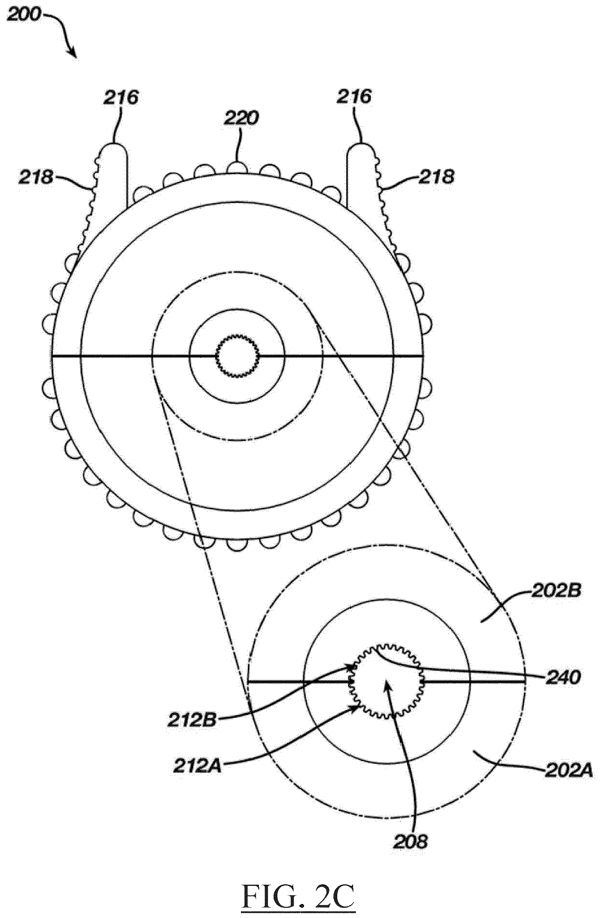

As illustrated in FIG. 2C, the first recess 212A and the second recess 212B can each have a textured inner surface configured to engage a catheter shaft 16. The textured inner surface can include a plurality of ribs 240 extending along the inner surface. Alternatively, or in addition, the textured inner surface can include a knurled finish and/or coined edge finish.

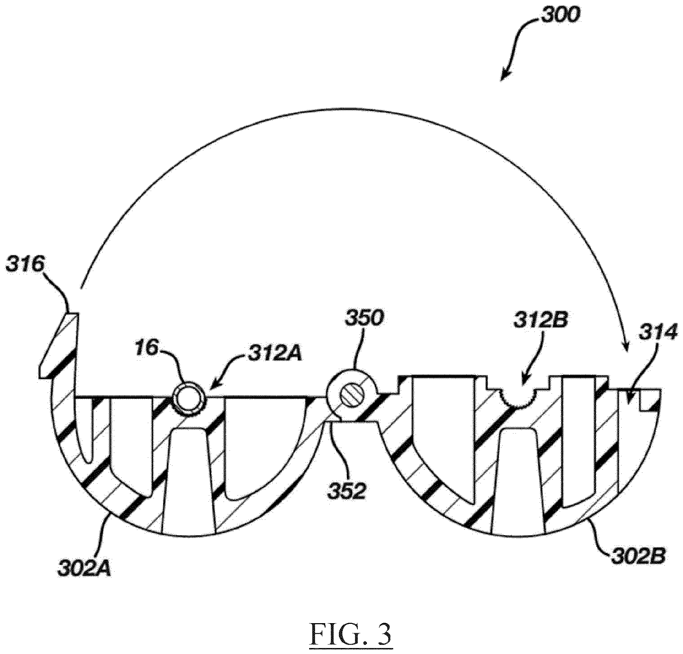

FIG. 3 is an illustration of a catheter shaft grip apparatus 300, in accordance with an example of the present invention. Catheter shaft grip apparatus 300 can include a first handle portion 302A and a second handle portion 302B. The first handle portion 302A and the second handle portion 302B can form the body of the catheter shaft grip apparatus 300. The first handle portion 302A can include a first recess 312A and the second handle portion 302B can include a second recess 312B. The first handle portion 302A and the second handle portion 302B can be pivotally connected such that the catheter shaft grip apparatus 300 can open and close. In the configuration shown in FIG. 3, the catheter shaft grip apparatus 300 is in an open or disengaged position. When catheter shaft grip apparatus 300 is in a closed or engaged position, the first recess 312A and the second recess 312B form an aperture extending along a central axis of the catheter shaft grip apparatus 300. The aperture can be the same or similar to the catheter shaft grip aperture 108 discussed above. For example, the aperture can be configured to frictionally engage the catheter shaft 16 such that when rotational force is applied to the body of the catheter shaft grip apparatus 300, the rotational force is transferred to the catheter shaft 16.

The first handle portion 302A and second handle portion 302B can be pivotally connected by a hinge. For example, the first handle portion 302A can include a first hinge portion 352 and the second handle portion 302B can include a second hinge portion 350. The first hinge portion 352 and second hinge portion 350 can be any hinge including, but not limited to, a living hinge, pin hinge, a snap hinge, etc. The first hinge portion 352 can be rotatably connected to the second hinge portion 350. As such, the first handle portion 302A and second handle portion 302B can be rotatably coupled and can be rotated between an open or disengaged position and a closed or engaged position. As will be appreciated, the aperture will be formed by the first recess 312A and the second recess 312B when the catheter shaft grip apparatus 300 is in the closed or engaged position. As such, the aperture can frictionally engage the catheter shaft 16 when the catheter shaft grip apparatus 300 is in the closed or engaged position.

The first handle portion 302A can further include a male connector 316 disposed on a side of the first handle portion 302A opposite the first hinge portion 352. The second handle portion 302B can include a female connector 314 disposed on a side of the second handle portion 302B opposite the second hinge portion 350. The female connector 314 can be configured to receive the male connector 316 and secure the first handle portion 302A to the second handle portion 302B when in the closed or engaged configuration. For example, the female connector 314 and the male connector 316 can form a snap-fit connection when in the closed or engaged configuration. Alternatively, or in addition, the female connector 314 and the male connector 316 can form a connection using a different mechanism such as tabs, screws, adhesive, hook-and-loop fasteners, interference fit, and the like, or any combination thereof.

As will be appreciated, the catheter shaft grip apparatus 300 can be configured in the same or similar manner to the catheter shaft grip apparatus 200 with the exception of how the connectors of each apparatus are configured (e.g., the one-piece hinged configuration of catheter shaft grip apparatus 300 and the two-piece configuration of catheter shaft grip apparatus 200). As such, the catheter shaft grip apparatus 300 can function in the same or similar manner to the catheter shaft grip apparatus 200 as described herein.

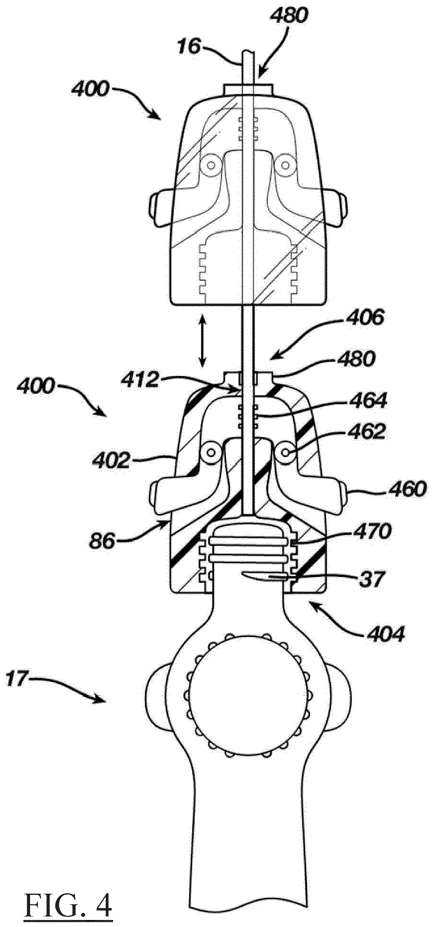

FIG. 4 is an illustration of a catheter shaft grip apparatus 400, in accordance with an example of the present invention. Catheter shaft grip apparatus 400 can include a body 402. The body 402 of the catheter shaft grip apparatus 400 can be disposed around a catheter shaft 16. For example, the catheter shaft grip apparatus 400 can have a catheter shaft grip aperture sized to receive a catheter shaft 16. The catheter shaft grip aperture can run along generally the central axis of the catheter shaft grip apparatus 400 from a proximal end 404 to a distal end 406. The catheter shaft grip apparatus 400 can include a proximal attachment end 470 configured to attach to a handle device 17 of a catheter 14. For example, the proximal attachment end 470 can be threaded and configured to attach to a threaded attachment end 37 of the handle device 17. As such, the catheter shaft grip apparatus 400 can be attached and detached to the handle device 17 by screwing and unscrewing the catheter shaft grip apparatus 400. As illustrated in FIG. 4, when the catheter shaft grip apparatus 400 is detached from the handle device 17, the catheter shaft grip apparatus 400 can be moved along the catheter shaft 16 away from the handle device 17. The catheter shaft grip apparatus 400 can include a distal attachment end 480 configured to attach to further devices. For example, the distal attachment end 480 can be configured to attach to a valve of a catheter assembly.

Catheter shaft grip apparatus 400 can further include a retention member configured to apply a force to the catheter shaft 16. For example, the retention member can include one or more lever arms 460. The lever arm 460 can include a hinge 462 and an engagement end 464. The lever arm 460 can be configured to actuate between an engaged position wherein the engagement end 464 applies a force to the catheter shaft 16 to a disengaged position wherein the engagement end 464 does not apply a force to the catheter shaft 16 or applies less of a force to the catheter shaft 16. For example, a user can push on an end of the lever arm 460 that is opposite the engagement end 464 and cause the lever arm 460 to pivot about the hinge 462 to move between the engaged position and disengaged position. A portion of the lever arm 460 disposed near the end of the lever arm 460 that is opposite the engagement end 464 can move into a gap 86 causing the engagement end 464 to move away from the catheter shaft 16. The retention member can be biased to remain in the engaged position. For example, the retention member can be spring-loaded such that the force exerted by the spring passively keeps the engagement end 464 pressed against the catheter shaft 16 until a user pushes on the lever arm 460 to disengage the engagement end 464. As will be appreciated, in the engagement position, the engagement end 464 of the retention member can frictionally engage the catheter shaft 16 such that when a rotational force is applied to the body 402 of the catheter shaft grip apparatus 400, the rotational force is transferred to the catheter shaft 16. Further, in the disengaged position the catheter shaft grip apparatus 400 can be freely movable along a catheter shaft 16. The engagement end 464 can include a textured surface configured to engage a catheter shaft 16. The textured surface can include a plurality of ribs extending along the textured surface. Alternatively, or in addition, the textured surface can include a knurled finish and/or coined edge finish.

As will be appreciated, the catheter shaft grip apparatus 400 can be configured in generally the same or similar manner to the catheter shaft grip apparatuses 200 and 300 with the exception of how the bodies of each apparatus are configured (e.g., the one piece installed on catheter shaft apparatus 400, the one-piece hinged configuration of catheter shaft grip apparatus 300, and the two-piece configuration of catheter shaft grip apparatus 200). As such, the catheter shaft grip apparatus 300 can function in the same or similar manner to the catheter shaft grip apparatus 200 as described herein.

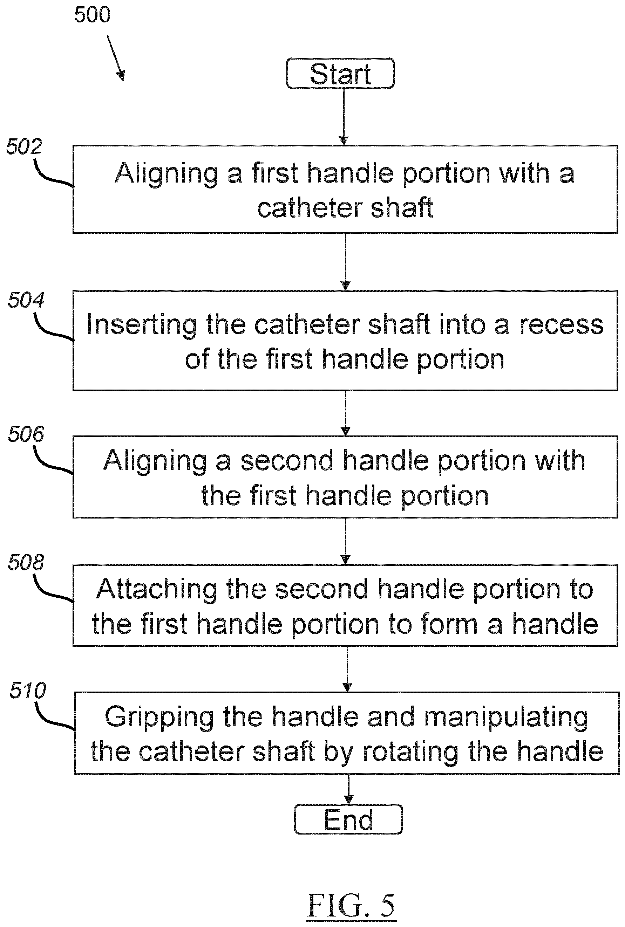

The disclosed technology includes methods of manipulating a catheter shaft using an attachable handle, such as method 500, which is illustrated in FIG. 5. Method 500 can include aligning 502 a first handle portion with a catheter shaft. For example, the first handle portion can be a first handle portion such as 202A or 303A of the catheter shaft grip apparatuses 200 and 300 described herein. The first recess 212A or 312A can be aligned with the catheter shaft, such as catheter shaft 16.

The method 500 can include inserting 504 a catheter shaft into a first recess of the first handle portion. For example, the catheter shaft (e.g., catheter shaft 16) can be inserted into the first recess (e.g., first recess 212A, 312A) of the catheter shaft grip apparatuses (e.g., catheter shaft grip apparatus 200, 300). The first recess can extend along a central axis of the first handle portion. For example, the first recess can extend from a proximal end to a distal end of the first handle portion.

The method 500 can include aligning 506 a second handle portion with the first handle portion. For example, the second handle portion can be a second handle portion 202B or 302B of the catheter shaft grip apparatuses 200 and 300 described herein. The first and second handle can include one or more alignment mechanisms for guiding the alignment of the first handle portion and the second handle portion. For example, the first handle portion and second handle portion can include one or more connectors that align the first handle portion and second handle portion when connected. The one or more connectors can be male and female connectors such as female connectors 214, 314 and male connectors 216, 316 of the catheter shaft grip apparatuses 200 and 300 described herein. Alternatively, or in addition, the first handle portion and second handle portion can include one or more protrusions and one or more recesses configured to receive the one or more protrusions such that when the recess receives the protrusion, the first handle portion and second handle portion are aligned. For example, the one or more protrusions can be protrusions 234 and the one or more recesses can be recesses 236 of the catheter shaft grip apparatus 200 described herein. The second handle portion can include a second recess that is aligned with the first recess of the first handle portion. For example, the second recess can be the second recess 212B, 312B of the catheter shaft grip apparatuses 200, 300 described herein. The second recess can extend along a central axis of the second handle portion. For example, the second recess can extend from a proximal end to a distal end of the second handle portion.

The method 500 can include attaching 508 the second handle portion to the first handle portion to form a handle. For example, the first handle portion and second handle portion can have one or more connectors designed to connect or mate with one another to attach the first handle portion to the second handle portion. The one or more connectors can be male and female connectors such as female connectors 214, 314 and male connectors 216, 316 of the catheter shaft grip apparatuses 200 and 300 described herein. When attached, the aligned first recess of the first handle portion and second recess of the second handle portion can form an aperture running through the handle. The aperture can be sized to receive a catheter shaft (e.g., catheter shaft 16).

The method 500 can include gripping 510 the handle and manipulating a catheter shaft by rotating the handle. For example, the aperture of the handle can frictionally engage a catheter shaft and transfers rotational force applied to the body to a catheter shaft. As such, when a user, such as a physician 24, grips the handle and rotates the handle, the catheter shaft will rotate.

Catheter shaft apparatuses disclosed herein, such as catheter shaft grip apparatus 200, 300, 400, can be installed to a shaft of a catheter to increase the grip diameter. The increased grip diameter can help users grip the catheter shaft apparatus, increase comfort, reduce fatigue and/or cramping, and increase fine control over the catheter. As will be appreciated, the increased grip diameter can reduce the force required to twist a catheter. For example, the catheter shaft apparatus can have a diameter of 1 inch which can provide an 89% reduction in required grip force when compared to a 0.105 inch catheter (e.g., an 8F catheter). Table 1 provides an example calculation. The limiting factor in this situation is the hand slipping on the shaft surface. Torque on a cylinder is exerted by a shearing (frictional) action on the cylinder's surface. Torque may be calculated using the following formula (Equation 1):

$$\text{Equation 1: } T = G \mu D$$

An estimated torque required to rotate the catheter is 1 in-lb of force. And with a dry glove, an estimated coefficient of static friction can be approximately 0.60. If gloves were wet the amount of hand force would increase. Finally, an 8F Catheter = 2.667 mm or .105" and a proposed diameter of the catheter shaft apparatuses disclosed herein is 1 inch. This proposed diameter of 1 inch is not meant to be limiting and the catheter shaft apparatus diameter can be larger than 1 inch or smaller than 1 inch. For example, larger and smaller size catheter shaft apparatuses can be provided to better fit a user's hand size, the catheter it is being used with, the rotational force required for a specific procedure, or the fine control needed for a specific procedure.

Table 1

| T (in-lb) | Grip force (linear force) | Friction Coefficient ($\mu$) | Diameter |
|---|---|---|---|
| 1 | 15.873 | 0.6 | 0.105" |
| 1 | 1.667 | 0.6 | 1" |

The catheter shaft apparatuses can make rotating a catheter shaft comfortable for almost all users. An estimated maximum force for the 50th percentile for a thumb-index pinch is 9.6 lbs. and 15.3 lbs. for women and men respectively. A comfortable force can be estimated as 1/3 of the maximum force. That is, a comfortable force can be estimated as 3.2 lbs. and 5.1 lbs. for women and men respectively. The estimated grip force of 1.667 lbs. required for the catheter shaft apparatuses, calculated above (see Table 1), is well below the estimated comfortable force.

The outer surface of the catheter shaft apparatuses can additionally include a textured surface, such as ribs, knurling, or a coined edge to help users grip the catheter shaft apparatus, increase comfort, reduce fatigue and/or cramping, and increase fine control over the catheter. As will be appreciated, the textured surface would further reduce the required grip force by increasing the friction coefficient. Further the outer shape of the catheter shaft apparatus can have an ergonomic shaft to additionally help users grip the catheter shaft apparatus, increase comfort, reduce fatigue and/or cramping, and increase fine control over the catheter.

The catheter shaft grip apparatuses can additionally include a tip disposed at the distal end configured to serve as an insertion tool. Alternatively, or in addition, the catheter shaft grip apparatuses can be used as an insertion depth limiter.

The disclosed technology described herein can be further understood according to the following clauses:

Clause 1: An apparatus to grip a catheter shaft, the apparatus comprising: a body including a proximal end and extending along a central axis to a distal end, and comprising: a first handle portion defining a first recess extending along the central axis of the body from the proximal end to the distal end; a second handle portion defining a second recess extending along the central axis of the body from the proximal end to the distal end such that the first recess is aligned with the second recess, and a catheter shaft grip aperture extending along the central axis of the body from the proximal end to the distal end, formed from the alignment of the first recess and the second recess, the catheter shaft grip aperture being sized to receive a catheter shaft.

Clause 2: The apparatus of Clause 1, wherein the catheter shaft grip aperture frictionally engages a catheter shaft and transfers rotational force applied to the body to a catheter shaft.

Clause 3: The apparatus of Clauses 1 or 2, the catheter shaft grip aperture comprising: a relaxed state where the catheter shaft grip aperture receives a catheter shaft and is freely movable along a catheter shaft; and a grip state, where a force applied to the body causes the catheter shaft grip aperture to frictionally engage a catheter shaft.

Clause 4: The apparatus of any preceding clause, the first handle portion comprising a first connector extending outwardly from the first handle portion, and the second handle portion comprising a second connector configured to receive the first connector and attach the first handle portion and the second handle portion to each other.

Clause 5: The apparatus of Clause 4, wherein the first connector comprises a first male connector disposed proximate the proximal end and the second connector comprises a first female connector disposed proximate the proximal end, wherein the first handle portion further comprises a second male connector extending outwardly from the first handle portion and disposed proximate the distal end, and wherein the second handle portion further comprises a second female connector disposed proximate the distal end and configured to receive the second male connector to attach the first handle portion and the second handle portion to each other.

Clause 6: The apparatus of Clause 1, the first handle portion including a first hinge portion and the second handle portion including a second hinge portion, the first hinge portion and the second hinge portion rotatably connected to each other such that the first handle portion and the second handle portion can be rotated between an engaged configuration and a disengaged configuration.

Clause 7: The apparatus of Clause 6, the first handle portion further comprising a male connector disposed on a side of the first handle portion opposite the first hinge portion, the second handle portion further comprising a female connector disposed on a side of the second handle portion opposite the second hinge portion such that the female connector is configured to receive the male connector and secure the first handle portion to the second handle portion when in the engaged configuration.

Clause 8: The apparatus of Clause 1, the first handle portion further comprising a second recess extending into the first handle portion generally perpendicularly to the central axis; and the second handle portion further comprising a protrusion extending outwardly from second handle portion generally perpendicularly to the central axis, wherein the second recess is configured to receive the protrusion to facilitate alignment of the first handle portion with the second handle portion.

Clause 9: The apparatus of claim 1 further comprising a retention member configured to apply a force to a catheter shaft when the retention member is in an engaged position.

Clause 10: The apparatus of Clause 9, wherein the retention member is rotatable between the engaged position and a disengaged position.

Clause 11: The apparatus of Clause 10, wherein the retention member comprises a lever arm including an engagement end configured to engage a catheter shaft, and wherein the lever arm is configured to be actuated between the engaged position and the disengaged position by a user pushing on the lever arm at an end opposite the engagement end.

Clause 12: The apparatus of any of Clauses 9-11, wherein the retention member is biased to remain in the engaged position.

Clause 13: The apparatus of any of Clauses 9-12, wherein the retention member is spring-loaded.

Clause 14: The apparatus of any of the preceding clauses, wherein the body further comprises a proximal attachment end configured to attach to a handle device of a catheter assembly.

Clause 15: The apparatus of Clause 14, wherein the attachment end comprises a threaded end.

Clause 16: The apparatus of any of the preceding clauses, wherein the body further comprises a distal attachment end configured to attach to a valve of a catheter assembly.

Clause 17: The apparatus of any of the preceding clauses, the first recess and the second recess each including a textured inner surface configured to engage a catheter shaft.

Clause 18: The apparatus of Clause 17, wherein the textured inner surface comprises a plurality of ribs extending along the inner surface.

Clause 19: The apparatus of Clause 17, wherein the textured inner surface comprises a knurled finish.

Clause 20: The apparatus of any of the preceding clauses, the body comprising a textured outer surface configured to be gripped by a user of the apparatus.

Clause 21: The apparatus of Clause 20, wherein the textured outer surface comprises a plurality of ribs extending along the outer surface.

Clause 22: The apparatus of Clause 20, wherein the textured outer surface comprises a knurled finish.

Clause 23: The apparatus of any of the preceding clauses, wherein the body includes a diameter of approximately one inch.

Clause 24: The apparatus of any of the preceding clauses, wherein the aperture comprises a diameter of approximately 0.1 inches.

Clause 25: The apparatus of any of the preceding clauses, wherein an inner diameter of the aperture is less than an outer diameter of a catheter shaft.

Clause 26: The apparatus of any of the preceding clauses, wherein the aperture forms a friction fit with a catheter shaft.

Clause 27: The apparatus of any of the preceding clauses, wherein the body comprises an ergonomic outer shape configured to conform to a user's hand.

Clause 28: The apparatus of any of the preceding clauses, wherein the body further comprises a tip proximate the distal end configured to serve as an insertion tool.

Clause 29: A handle for gripping a catheter shaft, the handle comprising: a first handle portion defining a first channel extending from a proximal end to a distal end of the first handle portion; and a second handle portion defining a second channel extending from a proximal end to a distal end of the second handle portion, the first handle portion and the second handle portion attachable to each other to form a handle including an aperture extending therethrough formed by the first channel and the second channel, the aperture sized to receive a catheter shaft.

Clause 30: The handle of Clause 29, the first handle portion comprising a first connector extending outwardly from the first handle portion, and the second handle portion comprising a second connector configured to receive the first connector and attach the first handle portion and the second handle portion to each other.

Clause 31: The handle of Clause 30, wherein the first connector comprises a first male connector disposed proximate the proximal end of the first handle portion and the second connector comprises a first female connector disposed proximate the proximal end of the second handle portion, wherein the first handle portion further comprises a second male connector extending outwardly from the first handle portion and disposed proximate the distal end of the first handle portion, and wherein the second handle portion further comprises a second female connector disposed proximate the distal end of the second handle portion and configured to receive the second male connector to attach the first handle portion and the second handle portion to each other.

Clause 32: The handle of Clause 31, the first handle portion further comprising a second recess extending into the first handle portion generally perpendicularly to a central axis of the first handle portion; and the second handle portion further comprising a protrusion extending outwardly from second handle portion generally perpendicularly to a central axis of the second handle portion, wherein the second recess is configured to at least partially receive the protrusion to facilitate alignment of the first handle portion with the second handle portion.

Clause 33: The handle of Clause 30, the first handle portion including a first hinge portion and the second handle portion including a second hinge portion, the first hinge portion and the second hinge portion rotatably connected to each other such that the first handle portion and the second handle portion can be rotated between an engaged configuration and a disengaged configuration.

Clause 34: The handle of Clause 33, wherein the first connector is disposed on a side of the first handle portion opposite the first hinge portion, and wherein the second connector disposed on a side of the second handle portion opposite the second hinge portion such that the second connector is configured to receive the first connector and secure the first handle portion to the second handle portion when in the engaged configuration.

Clause 35: A method of manipulating a catheter shaft using an attachable handle, the method comprising: aligning a first handle portion with a catheter shaft; inserting a catheter shaft into a first recess of the first handle portion, the first recess extending along a central axis of the first handle portion; aligning a second handle portion with the first handle portion; attaching the second handle portion to the first handle portion to form a handle, the second handle portion including a second recess extending along a central axis of the second handle portion such that the first recess and the second recess form an aperture through the handle, the aperture being sized to receive a catheter shaft; and gripping the handle and manipulating a catheter shaft by rotating the handle.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

**Claims**

1. An apparatus to grip a catheter shaft, the apparatus comprising:
   a body including a proximal end and extending along a central axis to a distal end, and comprising:

      a first handle portion defining a first recess extending along the central axis of the body from the proximal end to the distal end;
      a second handle portion defining a second recess extending along the central axis of the body from the proximal end to the distal end such that the first recess is aligned with the second recess, and
      a catheter shaft grip aperture extending along the central axis of the body from the proximal end to the distal end, formed from the alignment of the first recess and the second recess, the catheter shaft grip aperture being sized to receive a catheter shaft.

2. The apparatus of claim 1, wherein the catheter shaft grip aperture frictionally engages a catheter shaft and transfers rotational force applied to the body to a catheter shaft.

3. The apparatus of claim 1, the catheter shaft grip aperture comprising:

a relaxed state where the catheter shaft grip aperture receives a catheter shaft and is freely movable along a catheter shaft; and

a grip state, where a force applied to the body causes the catheter shaft grip aperture to frictionally engage a catheter shaft.

4. The apparatus of claim 1, the first handle portion comprising a first connector extending outwardly from the first handle portion, and

the second handle portion comprising a second connector configured to receive the first connector and attach the first handle portion and the second handle portion to each other, optionally wherein the first connector comprises a first male connector disposed proximate the proximal end and the second connector comprises a first female connector disposed proximate the proximal end,

wherein the first handle portion further comprises a second male connector extending outwardly from the first handle portion and disposed proximate the distal end, and

wherein the second handle portion further comprises a second female connector disposed proximate the distal end and configured to receive the second male connector to attach the first handle portion and the second handle portion to each other.

5. The apparatus of claim 1, the first handle portion including a first hinge portion and the second handle portion including a second hinge portion, the first hinge portion and the second hinge portion rotatably connected to each other such that the first handle portion and the second handle portion can be rotated between an engaged configuration and a disengaged configuration.

6. The apparatus of claim 5, the first handle portion further comprising a male connector disposed on a side of the first handle portion opposite the first hinge portion,

the second handle portion further comprising a female connector disposed on a side of the second handle portion opposite the second hinge portion such that the female connector is configured to receive the male connector and secure the first handle portion to the second handle portion when in the engaged configuration.

7. The apparatus of claim 1, the first handle portion further comprising a second recess extending into the first handle portion generally perpendicularly to the central axis; and

the second handle portion further comprising a protrusion extending outwardly from second handle portion generally perpendicularly to the central axis,

wherein the second recess is configured to receive the protrusion to facilitate alignment of the first handle portion with the second handle portion.

8. The apparatus of claim 1 further comprising a retention member configured to apply a force to a catheter shaft when the retention member is in an engaged position.

9. The apparatus of claim 8, wherein the retention member is rotatable between the engaged position and a disengaged position.

10. The apparatus of claim 9, wherein the retention member comprises a lever arm including an engagement end configured to engage a catheter shaft, and

wherein the lever arm is configured to be actuated between the engaged position and the disengaged position by a user pushing on the lever arm at an end opposite the engagement end.

11. The apparatus of claim 9, wherein the retention member is biased to remain in the engaged position, optionally wherein the retention member is spring-loaded.

12. The apparatus of claim 1, wherein the body further comprises a proximal attachment end configured to attach to a handle device of a catheter assembly, and/or a distal attachment end configured to attach to a valve of a catheter assembly, and/or a tip proximate the distal end configured to serve as an insertion tool.

13. A handle for gripping a catheter shaft, the handle comprising:

a first handle portion defining a first channel extending from a proximal end to a distal end of the first handle portion; and

a second handle portion defining a second channel extending from a proximal end to a distal end of the second handle portion, the first handle portion and the second handle portion attachable to each other to form a handle including an aperture extending therethrough formed by the first channel and the second channel, the aperture sized to receive a catheter shaft.

14. The handle of claim 13, the first handle portion comprising a first connector extending outwardly from the first handle portion, and

the second handle portion comprising a second connector configured to receive the first connector and attach the first handle portion and the second handle portion to each other, optionally wherein the first connector comprises a first male connector disposed proximate the proximal end of the first handle portion and the second connector comprises a first female connector disposed proximate the proximal end of the second handle portion, wherein the first handle portion further comprises a second male connector extending outwardly from the first handle portion and disposed proximate the distal end of the first handle portion, and wherein the second handle portion further comprises a second female connector disposed proximate the distal end of the second handle portion and configured to receive the second male connector to attach the first handle portion and the second handle portion to each other.

15. A method of manipulating a catheter shaft using an attachable handle, the method comprising:

aligning a first handle portion with a catheter shaft;
inserting a catheter shaft into a first recess of the first handle portion, the first recess extending along a central axis of the first handle portion;
aligning a second handle portion with the first handle portion;
attaching the second handle portion to the first handle portion to form a handle, the second handle portion including a second recess extending along a central axis of the second handle portion such that the first recess and the second recess form an aperture through the handle, the aperture being sized to receive a catheter shaft; and
gripping the handle and manipulating a catheter shaft by rotating the handle.

**FIG. 1**

EP 4 382 158 A1

FIG. 2A

FIG. 2B

FIG. 2C

FIG. 3

FIG. 4

EP 4 382 158 A1

500

```
                          ┌──────────┐
                          │  Start   │
                          └──────────┘
                                │
                                ▼
502   ┌─────────────────────────────────────────────┐
      │   Aligning a first handle portion with a      │
      │              catheter shaft                   │
      └─────────────────────────────────────────────┘
                                │
                                ▼
504   ┌─────────────────────────────────────────────┐
      │  Inserting the catheter shaft into a recess   │
      │         of the first handle portion           │
      └─────────────────────────────────────────────┘
                                │
                                ▼
506   ┌─────────────────────────────────────────────┐
      │   Aligning a second handle portion with       │
      │           the first handle portion            │
      └─────────────────────────────────────────────┘
                                │
                                ▼
508   ┌─────────────────────────────────────────────┐
      │  Attaching the second handle portion to       │
      │  the first handle portion to form a handle    │
      └─────────────────────────────────────────────┘
                                │
                                ▼
510   ┌─────────────────────────────────────────────┐
      │   Gripping the handle and manipulating        │
      │  the catheter shaft by rotating the handle    │
      └─────────────────────────────────────────────┘
                                │
                                ▼
                          ┌──────────┐
                          │   End    │
                          └──────────┘
```

FIG. 5

22

Europäisches
Patentamt

European
Patent Office

Office européen
des brevets

# EUROPEAN SEARCH REPORT

**Application Number**

EP 23 21 3865

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/238829 A1 (PARKER STEVEN [US]) 24 August 2017 (2017-08-24) * page 1, paragraph 2 - page 3, paragraph 30; figures 1A-5D * | 1-10, 12-15 | INV. A61M25/01 |
| X | US 2018/339136 A1 (WOOD ZACHARY [US] ET AL) 29 November 2018 (2018-11-29) * page 1, paragraph 2 - page 9, paragraph 82; figures 1-30 * | 1-3,5, 7-15 | |
| X | EP 2 875 842 A1 (FIAB S P A [IT]) 27 May 2015 (2015-05-27) * column 1, line 3 - column 16, line 10; figures 1A-7C * | 1-15 | |
| X | EP 0 567 029 A1 (BECTON DICKINSON CO [US]) 27 October 1993 (1993-10-27) * column 1, line 3 - column 5, line 6; figures 1-9 * | 1-15 | |
| X | WO 96/02294 A1 (HWANG CHIN RONG [BR]) 1 February 1996 (1996-02-01) * page 1, line 3 - page 4, line 11; figures 1-12 * | 1-15 | **TECHNICAL FIELDS SEARCHED (IPC)** A61M |
| X | GB 2 520 332 A (MEDITECH ENDOSCOPY LTD [GB]) 20 May 2015 (2015-05-20) * page 1, line 7 - page 22, line 29; figures 1-15 * | 1-15 | |

The present search report has been drawn up for all claims

| Place of search | Date of completion of the search | Examiner |
|---|---|---|
| Berlin | 24 April 2024 | Rolland, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 3865

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2024

| Patent document cited in search report | Publication date | Patent family member(s) | Publication date |
|---|---|---|---|
| US 2017238829 A1 | 24-08-2017 | NONE | |
| US 2018339136 A1 | 29-11-2018 | NONE | |
| EP 2875842 A1 | 27-05-2015 | EP 2875842 A1 | 27-05-2015 |
| | | US 2015141914 A1 | 21-05-2015 |
| EP 0567029 A1 | 27-10-1993 | AU 3701293 A | 28-10-1993 |
| | | CA 2094105 A1 | 25-10-1993 |
| | | DE 69308358 T2 | 12-06-1997 |
| | | EP 0567029 A1 | 27-10-1993 |
| | | JP 2575277 B2 | 22-01-1997 |
| | | JP H0663153 A | 08-03-1994 |
| | | US 5382239 A | 17-01-1995 |
| WO 9602294 A1 | 01-02-1996 | BR 7401236 U | 19-03-1996 |
| | | WO 9602294 A1 | 01-02-1996 |
| GB 2520332 A | 20-05-2015 | AU 2014349856 A1 | 16-06-2016 |
| | | CA 2930746 A1 | 21-05-2015 |
| | | EP 3071089 A2 | 28-09-2016 |
| | | ES 2747637 T3 | 11-03-2020 |
| | | GB 2520332 A | 20-05-2015 |
| | | GB 2520376 A | 20-05-2015 |
| | | JP 6465896 B2 | 06-02-2019 |
| | | JP 2016537169 A | 01-12-2016 |
| | | US 2016296105 A1 | 13-10-2016 |
| | | WO 2015071688 A2 | 21-05-2015 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5391199 A
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B